# EUROPEAN PATENT SPECIFICATION

(11) **EP 2 272 507 B1**
(45) Date of publication and mention of the grant of the patent: **10.10.2012**
(21) Application number: 10012336.3
(22) Date of filing: 26.07.2004
(51) Int. Cl.: A61K 9/70, A61K 31/485

(54) **Transdermal formulation comprising an opioid analgesic and an aloe composition**
Trandermale Formulierung mit einem opioiden Analgetikum und einer Aloe-Zusammensetzung
Formulation pour administration transdermique comprenant un analgésique opioide et une composition d'aloès

(30) Priority: 02.09.2003 DE 10340428
(43) Date of publication of application: 12.01.2011
(62) Divisional of application: 04763497.7
(73) Proprietor: Acino AG, 83714 Miesbach (DE)
(72) Inventor: Meyer, Elisabeth, 83714 Miesbach (DE); Altenschöpfer, Peter, 83677 Greiling (DE); Baumann, Angelika, 83714 Miesbach (DE); McLeod, Sarah, 81675 München (DE)
(74) Representative: Beetz & Partner

(56) References cited:
- WO-A-03/079945
- US-B1- 6 455 066

## Description

### FIELD OF THE INVENTION

The invention relates to methods and compositions for transdermal administration of analgesics with the aid of a transdermal penetration agent.

### BACKGROUND OF THE INVENTION

Transdermal drug application offers distinct advantages over conventional administration methods. For example, some drugs cannot be absorbed from the digestive tract, and intravenous and subcutaneous administration by injection is painful and invasive. Another reason to to avoid systemic firstpass metabolism upon oral administration.

Unfortunately, because of the skin's drug penetration resistance, only a limited number of drugs are bioavailable via transdermal application (Ghosh, T. K.; Pfister, W. R.) Yum, S. I., Transdermal and Topical Drug Delivery Systems, Interpharm Press, Inc. p. 7), The skin is a complex multilayer organ with a total thickness of 2-3 mm. The panniculus adiposus, a variably thick fatty layer, is below the dermis. The dermis is a layer of dense connective tissue that supports the epidermis. The epidermis comprises a layer of epithelial cells and is about 100 µm thick. The epidermis is further classified into a number of layers, of which the outermost layer is the stratum corneum (15-20 µm thick). The stratum corneum comprises highly dense, keratinized tissue and is the skin's main source of penetration and permeation resistance (Montagna, W. and Parakkal, P. F. (1974) The Structure and Function of Skin, Academic Press, New York, and Holbrook, K. A. and Wolf, K. (1993), The Structure and Development of Skin, In: Dermatology in General Medicine, Vol 1, 4th ed., Eds. T. B. Fitzpatrick, A. Z. Eisen, K. Wolff, I. M. Feedberg, and K. F. Austen, McGraw Hill, Inc., New York, pp. 97-145).

The following sequence of mechanisms has been proposed for transdermal absorption: 1) partitioning of the drug from the applied vehicle into the stratum corneum; 2) diffusion through the stratum corneum; 3) partitioning from the stratum into the epidermis; 4) diffusion through the epidermis; and 5) capillary uptake (Potts et al. (1992) Percutaneous Absorption: Pharmacology of the Skin, Ed. Mukhtar, H. CRC Press, pp. 13-27).

Because of the skin's intrinsic resistance to drug penetration, penetration agents are often essential to assist transdermal drug administration. The term penetration agent has generally been applied to the class of chemicals that increase the partitioning and/or diffusion of the active agent. (for example see, Ghosh, T. K. et al. (1993), Pharm. Tech. 17(3):72-98; Ghosh, T. K. et al. (1993), Pharm. Tech. 17(4): 62-89; Ghosh, T. K. et al. (1993), Pharm. Tech. 17(5) : 168-76; Pfister et al. Pharm. Tech. 14(9) :132-140). Ideally penetration agents should be pharmacologically inert, non-toxic, non-irritating and non-allergenic, compatible with the formulation components, have rapid onset of action, and be reversible in their reduction of skin-barrier properties. The penetration agent should also spread well on the skin with a suitable skin feel. In practice, all of these ideal requirements are rarely met, and a need exists for improved penetration agents. (Aungst (1991), skin Permeation Enhancers for Improved Transdermal Drug delivery. In: High Performance Biomaterial: A Comprehensive Guide to Medical and Pharmaceutical Applications, Ed. M. Szycher, pp. 527-538).

Aloe, a plant species native to Africa, is also known as "lily of the deaert", the "plant of immortality", and the "medicine plant". The name was derived from the Arabic alloeh meaning "bitter" because of the bitter liquid found in the leaves. In 1500 B.C. Egyptians recorded use of the herbal plant in treating burns, infection and parasites.

There are over 500 species of aloe growing in climates worldwide. Ancient Greeks, Arabs and Spaniards have used the plant throughout the millennia. African hunters still rub the gel on their bodies to reduce perspiration and their scent. Extensive research since the 1930'a has shown that the clear gel has a dramatic ability to heal wounds, ulcers and burns by putting a protective coating on the affected areas and speeding up the healing rate.

The plant is about 96% water. The rest of it contains active ingredients including essential oil, amino acids, minerals, vitamins, enzymes and glycoproteins. Modern healers have used it as medicinal plant since the 1930's. Many liquid health treatments are made, some combining aloe juice with other plants and herbs. The juice is soothing to digestive tract irritations, such as colitis and peptic ulcers.

Aloe contains at least three anti-inflammatory fatty acids that are helpful for the stomach, small intestine and colon. A newly discovered compound in aloe, acemannan, is currently being studied for its ability to strengthen the bodies natural resistance. Studies have shown acemannan to boost T-lymphocyte cells that aid the immune system.

In transdermal therapeutic systems, aloe vera is implemented generally as skin protectant. In US 6,455,066 aloe vera is described to increase penetration of Lidocain through the stratum corneum into the epidermis or dermis. Nevertheless, aloe vera did not influence absorption of the drug by blood capillaries. Surprisingly and for the first time, we observed a significant effect of aloe vera as a drug penetration enhancer for an analgesic substance through the skin including the absorption of the substance by a buffer system.

### SUMMARY OF THE INVENTION

The problem underlaying the invention is solved by a transdermal patch comprising an opioid analgesic from the phenanthrene group or a pharmaceutically acceptable salt thereof as active ingredient and an aloe composition as transdermal penetration agent and a backing, a pressure sensitive adhesive and a release liner, wherein the adhesive consists of or comprises a rubber adhesive selected from a styrene-butadiene-styrene block copolymer and a styrene-butadiene block copolymer.

The formulation according to the invention can be a patch provided with a covering layer.

Further, according to the invention the patch can be a formulation selected from the group of matrix type patch, reservoir type patch, multi-laminate drug-in-adhesive type patch, and monolithic drug-in-adhesive type patch.

Further, according to the invention the formulation can be a monolithic drug-in-adhesive type patch.

Further, according to the invention the adhesive can comprise or consist of a component selected from the group of natural rubber; synthetic rubber, acrylic adhesive; polyvinylacetate; polydimethylsiloxane; and hydrogels, especially high molecular weight polyvinlpyrrolidone and oligomeric polyethylene oxide.

Further, according to the invention the further adhesive can be acrylic adhesive.

Further, according to the invention the acrylic adhesive can comprise or consist of a polyacrylate,

Further, according to the invention the polyacrylate can be selected from the group consisting of polybutylacrylate, polmethylacrylate and poly-2-ethylhexylacrylate.

Further according to the invention the adhesive can contain a crosslinker.

Further, according to the invention the analgesic oan be buprenorphine or a pharmaceutically acceptable salt thereof.

Further, according to the invention the analgesic can be buprenorphine or a pharmaceutically acceptable salt thereof.

Further, according to the invention the extracting agent of the aloe extract or the vehicle can be a vegetable oil.

Further, according to the invention the vegetable oil can be a hydrogenated oil.

Further, according to the invention the vegetable oil can be soybean oil.

Further, according to the invention the formulation can comprise another penetration agent in addition to the aloe composition.

Further, according to the invention the additional penetration agent is selected from the group consisting of ethyl alcohol; isopropyl alcohol; octyl phenol; polyethylene glycol octylphenyl ether; oleic acid; poyethyline glycol (PEG), especially PEG 400; propylene glycol; N-decylmethyl sulfoxide; fatty acid esters, especially isopropyl myristate, methyl laurate, glycerol monooleate and propylene glycol monooleate; and N-methyl pyrrolidone.

Further, according to the invention the composition can comprise a preservative, especially a preservative selected from the group of alcohols, quaternary amines, organic acids, parabens and phenols.

Further, according to the invention the formulation can comprise a backing comprising or consisting of a material selected from the group consisting of polyolefin, polyester, polyvinylidene chloride, polyurethane, cotton or wool.

Further, according to the invention the backing can be a polyolefine foil.

Finally, according to the invention the foil can have a thickness of 0.5 to 1.5 and especially 0.6 to 1.0 mm.

From the foregoing description follows that it has now been found that aloe compositions are penetration agents for enhancing penetration of transdermally applied analgesics through the stratum corneum and into the epidermis or dermis and further into the circulating blood.

In one embodiment, the invention comprises a method for administering an analgesic. The method comprises applying to the subject's skin a pharmaceutically acceptable transdermal formulation comprising a therapeutically effective amount of the analgesic or a pharmaceutically acceptable salt thereof and a penetration enhancing amount of an transdermal penetration agent selected from the group consisting of an aloe composition, the pharmaceutically acceptable transdermal formulation being a patch.

In still another embodiment, the invention relates to a patch comprising a penetration enhancing amount of a transdermal penetration agent selected from the group consisting of an aloe composition, and a therapeutically effective amount of a analgesic to administer the analgesic in a subject in need of an analgesic effect packaged in association with instructions, the instructions comprising: applying the patch to the skin.

In yet another embodiment, the invention relates to a patch comprising a backing and a pressure sensitive styrene-butadiene-styrene adhesive, which adhesive comprises a therapeutically effective amount of an analgesic and a penetration enhancing amount of a transdermal penetration agent selected from an aloe composition.

These and other features, aspects, and advantages of the invention will become better understood with reference to the following detailed description, example, and appended claims.

### DETAILED DESCRIPTION OF THE INVENTION

In one embodiment, the invention comprises a method for administering an analgesic in a subject in need of an analgesic effect, applying to the subject's skin a pharmaceutically acceptable transdermal formulation in the form of a patch comprising a therapeutically effective amount of the analgesic or a pharmaceutically acceptable salt thereof and a penetration enhancing amount of a transdermal penetration agent selected from an aloe composition.

As used herein the term "transdermal penetration agent" means an agent capable of transporting a pharmacologically active compound through the stratum corneum into the epidermis or dermis, and a further capillary uptake. A "penetration enhancing amount" of a transdermal penetration agent is an amount which enhances the analgesic penetration rate through the stratum corneum and/or increases solubility of the analgesic within the skin.

The term "pharmaceutically acceptable transdermal formulation" as used herein means any formulation which is pharmaceutically acceptable for transdermal administration of an analgesic. According to the invention, a "transdermal formulation" will comprise at least an analgesic and a transdermal penetration agent. The choice of transdermal formulation will depend on several factors, including the condition to be treated, the physicochemical characteristics of the analgesic and other excipients present, their stability in the formulation, available manufacturing equipment, and costs constraints.

As used herein, a "therapeutically effective amount of an analgesic" means an amount of the analgesic required to induce an analgesic effect sufficient to suppress pain.

As used herein, the term "subject" means any animal, preferably a mammal, more preferably a human.

oily Aloe extract is particularly preferred for use with the invention, for example, but not limited to, nut oils, such as almond oil and walnut oil; castor oil; coconut oil; corn oil; cotton seed oil; jojoba oil; linseed oil; grape seed oil; rape seed oil; mustard oil; olive oil; palm and palm kernel oil; peanut oil; safflower oil; sesame oil; soybean oil; sunflowerseed oil; crambe oil; wheat germ oil; cocoa butter; or mixtures thereof as extractring agent or vehicle. Soybean oil is a preferred vegetable oil for use with the invention. If desired, the vegetable oils may be processed, for example by hydrogenation.

As used herein, the term "aloe composition" means any extract or processed form of a plant of the Genus Aloe, family Liliaceae. For example, aloe extracts and processed forms of aloe for use with the invention may be obtained from Aloe *arbrorescens,* Aloe *barbandensis,* or Aloe ferox species of aloe. Any part of the plant may be processes or extracted, such as the leaf, stem, or flower. Examples of suitable aloe compositions include Aloe *arbrorescens* leaf extract (Maruzen Pharmaceuticals, Morristown, N.J.); Aloe *barbandensis* leaf extract (Florida Food Products, Inc., Eustis, Fla.); Aloe *barbandensis* flower extract (Tri-K, Industries, Emerson, N.J.); Aloe *barbandensis* gel (Active Organics, Dallas, Tex.); and Aloe *ferox* leaf extract (Maruzen Pharmaceuticals, Morristown, N.J.). The preferred aloe composition for use with the invention is aloe barbandensis gel, which is the fresh mucilaginous gel obtained from the parenchymatous tissue in the leaf center, referred to herein as "aloe-vera gel".

As used herein, the term "analgesic" means any drug that provides analgesia or any drug that provides a blockage of nociceptive pain and in cases also neuropathic pain. The analgesic can be any opioid analgesic from the phenanthrene group, as for example Buprenorphine or Nalbuphine, and pharmaceutically acceptable salts thereof, or mixtures thereof.

Furthermore, in order to improve the effectiveness and tolerance of the present topically effective therapy, opioid analgesics with different pharmacodynamics and pharmacokinetics may be combined in a pharmaceutically acceptable topical formulation.

The phrase "pharmaceutically acceptable salt(s)", as used herein, unless otherwise indicated, means those salts that retain the biological effectiveness and properties of neutral analgesics and that are not otherwise unacceptable for pharmaceutical use. Pharmaceutically acceptable salts include salts of acidic or basic groups, which groups may be present in the opioid analgesics. Pharmaceutically acceptable addition of salts of basic opioid analgesics used in the present invention are those that form non-toxic salts, i.e., salts comprising pharmacologically acceptable anions, such as the hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate (i.e., 1,1'-methylene-bis-(2-hydroxy-3-naphthoate)) salts. The analgesics of the present invention that include an amino moiety may form pharmaceutically acceptable salts with various amino acids. Suitable base salts are formed from bases which form non-toxic salts and examples are aluminium, calcium, lithium, magnesium, potassium, sodium, zinc and diethanolamine salts. For a review on pharmaceutically acceptable salts see Berge et al., J. Pharm. Sci., 66, 1-19 (1977).

According to the present invention, any combination of an aloe composition, for example, a mixture of soybean oil and aloe-vera gel can be used.

Suitable preservatives include, but are not limited to, alcohols, quaternary amines, organic acids, parabens, and phenols.

suitable antioxidants include, but are not limited to, ascorbic aoid and its asters, sodium bisulfite, butylated hydroxytoluene, butylated hydroxyanisole, tocopherols, and chelating agents like EDTA and citric acid.

Suitable moisturizers include, but are not limited to, glycerine, sorbitol, polyethylene glycols, urea, and propylene glycol.

Suitable buffering agents for use with the invention include, but are not limited to, citric, hydrochloric, and lactic acid buffers.

Suitable solubilizing agents include, but are not limited to, quaternary ammonium chlorides, cyclodextrins, benzyl benzoate, lecithin, and polysorbates.

Penetration agents for use with the invention include, but are not limited to, ethyl alcohol, isopropyl alcohol, octolyphenylpolyethylene glycol, oleic avoid, polyethylene glycol 400, propylene glycol, N-decylmethyleulfoxide, fatty acid esters (e.g., isopropyl myristate, methyl laurate, glycerol monooleate, and propylene glycol monooleate); and N-methyl pyrrolidone.

Suitable skin protectants that can be used in the topical formulations of the invention include, but are not limited to, vitamin E oil, allatoin, dimethicone, glycerin, petrolatum, and zinc oxide.

The formulation comprising an analgesic and a transdermal penetration agent is container in a patch that is applied adjacent to the skin. As used herein a "patch" comprises at least a topical formulation and a covering layer, such that the patch can be placed over the skin. Preferably, the patch is designed to reduce lag time, promote uniform absorption, and reduce mechanical rub-off.

preferably, the patch components resemble the viscoelastic properties of the skin and conform to the skin during movement to prevent undue shear and delamination.

A patch comprising the topical formulation of the invention has advantages over conventional methods of administration. One advantage is that the dose is controlled by the patch's surface area. Other advantages of patches are constant rate of administration, longer duration of action (the ability of to adhere to the skin for 1, 3, 7 days or longer) ; improved patient compliance, non-invasive dosing, and reversible action (i.e., the patch can simply be removed).

Preferred patches include (1) the matrix type patch; (2) the reservoir type patch; (3) the multi-laminate drug-in-adhesive type patch; and (4) the monolithic drug-in-adhesive type patch (Ghosh, T. K.; Pfister, W. R.; Yum, S. I., Transdermal and Topical Drug Delivery Systems, Interpharm Press, Inc. p. 249-297). These patches are well known in the art and generally available commercially.

For practice of the invention, the matrix type and the drug-in-adhesive type patches are especially preferred. The more preferred drug-in-adhesive patch is the monolithic type.

The matrix patch comprises an analgesio containing matrix, an adhesive backing film overlay and a release liner. In some cases, it may be necessary to include an impermeable layer to minimize drug migration into the backing film (e.g., U.S. Pat. No. 4,336,243). The analgesic containing matrix is held against the skin by the adhesive overlay. Examples of suitable analgesic matrix materials include but are not limited to lipophilic polymers, such as polyvinylchloride, polydimethylsiloxane, and hydrophilic polymers like polyvinylpyrrolidone, polyvinyl alcohol, hydrogels based on gelatin, or polyvinylpyrrolidone/polyethylene oxide mixtures.

The reservoir type patch design is characterized by a backing film coated with an adhesive, and a reservoir compartment comprising a drug formulation preferably, in the form of a solution or suspension, that is separated from the skin by a semipermeable membrane (e.g., U.S. Pat. No. 4,615,699). The adhesive coated backing layer extends around the reservoir's boundaries to provide a concentric seal with the skin and hold the reservoir adjacent to the skin.

The monolithic drug-in-adhexive patch design is characterized by the inclusion of the drug formulation in the skin contacting adhesive layer, a backing film and preferably, a release liner. The adhesive functions both to release the analgesic and adhere the analgesic matrix to the skin. The drug-in-adhesive system does not require an adhesive overlay. Also, drug-in-adhesive type patches are thin and comfortable (e.g., U.S. Pat. No. 4,751,087).

The multi-laminate drug-in-adhesive patch design further incorporates an additional semi-permeable membrane between two distinct drug-in-adhesive layers or multiple drug-in-adhesive layers under a single backing film (Peterson, T. A. and Dreyer, S. J., Proceed. Intern. Symp. Control. Rel. Bioact. Mater. 21; 477-478).

Semi permeable membranes, useful with the reservoir or multi-laminate patch, include thin non-porous ethylene vinyl acetate films or thin microporous films of polyethylene employed in microlaminate solid state reservoir patches.

Adhesives for use with the drug-in-adhesive type patches are well known in the art and selection is readily accomplished by an ordinary practitioner. Three basic types commonly used are polyisobutylenes, silicones, and acrylics. Adhesives useful in the present invention can function under a wide range of conditions, such as, high and low humidity, bathing, sweating etc. The adhesive may be a composition based on natural or synthetic rubber; a polyacrylate such as, polybutylacrylate, polymethylacrylate, poly-2-ethylhexyl acrylate; polyvinylacetate; polydimethylsiloxane; or and hydrogels (e.g., high molecular weight polyvinylpyrrolidone and oligomeric polyethylene oxide). The most preferred adhesive is a pressure sensitive rubber styrene-butadiene-styrene copolymer adhesive, for example Durotak.RTM. adhesives (e.g., Durotak.RTM. 87-6173, National Starch and Chemicals). The adhesive may contain a thickener, such as a silica thickener (e.g., Aerosil, Degussa, Ridgefield Park, N.J.) or a crosslinker such as, aluminumacetylacetonate.

Suitable release liners include but are not limited to occlusive, opaque, or clear polyester films with a thin coating of pressure sensitive release liner (e.g., silicone-fluorsilicone, and perfluorcarbon based polymers.

Backing films may be occlusive or permeable and are derived from synthetic polymers like polyolefin oils polyester, polyethylene, polyvinylidine chloride, and polyurethane or from natural materials like cotton, wool, etc. Occlusive backing films, such as synthetic polyesters, result in hydration of the outer layers of the stratum corneum while non-occlusive backings allow the area to breath (i.e., promote water vapor transmission from the skin surface). More preferably the backing film is an occlusive polyolefin foil (Alevo, Dreieich; Germany). The polyolefin foil is preferably about 0.6 to about 1 mm thick.

The amount of analgesic in the topical formulation will generally be from about 0.5% to about 25% of the total weight of the formulation.

In general, transdermal penetration agent will comprise of from about 0.5 percent to about 40 percent by weight of the patch's total weight, preferably of from about 2 percent to about 20 percent.

Although the present invention has been described in considerable detail with reference to certain preferred embodiments, other embodiments are possible.

### EXAMPLES

The following examples are provided for illustrative purposes only and are not to be construed as limiting the invention's scope in any manner.

### Example 1

Comparative skin permeation study of different Buprenorphine/Aloe Vera compositions.

The study was conducted with a 1.05 cm² matrix patch. The matrix comprised a mixture of buprenorphine and aloe as a transdermal penetration agent in an styrene-butadiene-styrene polymer.

The matrix was prepared by dissolving buprenorphine (5-15 weight percent) in ethylmethylketone (ratio 1 : 4.2); adding the transdermal penetration agent (0-20 weight percent) and a styrene-butadiene-styrene polymer (Durotak,RTM, -6173), and mixing until homogeneous. The homogeneous mixture was then coated on a polyolefin foil with a hand-coater machine to an average area weight of about 50g/m². The coated foil was dried for about 15 min. at about 801C to evaporate the solvent ethylmethylketone. The amounts of buprenorphine and transdermal penetration agent in each patch are given in the Table I below as the percentage of the total patch weight.

The matrix patch was applied to female hairless mouse skin.

**TABLE I: Amounts of buprenorphine and aloe vera and corresponding flux data.**

| *batch* | *Buprenorphine* % | *aloe* % | *PSA* | *flux* *(hairless mouse skin)* |
|---|---|---|---|---|
| 001 | 15 | 20 | DT 6173 | 2,3 µg/(cm²*h) |
| 002 | 5 | 20 | DT 6173 | 0,8 µg/(cm²*h) |
| 003 | 10 | 10 | DT 6173 | 0,9 µg/(cm²*h) |

The flux data in Table I above demonstrate that the amount of aloe vera in the matrix efficiently impacts on the flux. Whereas a transdermal matrix system with only 10% of aloe leads to a max. flux of 0,9 µg/(cm²*h), a system containing 20% aloe vera (& 15% buprenorphine, see batch 001) shows a flux of up to 2,3 µg/(cm²*h).

The foregoing has outlined rather broadly the more pertinent and important features of the present invention.

## Claims

1. Transdermal patch comprising
- an opioid analgesic from the phenanthrene group or a pharmaceutically acceptable salt thereof as active ingredient,
- an aloe composition as transdermal penetration agent and
- a covering layer, a pressure sensitive adhesive and a release liner, wherein the adhesive consists of or comprises a rubber adhesive selected from a styrene-butadiene-styrene block copolymer and a styrene-butadiene block copolymer.

2. Transdermal patch according to claim 1, wherein the patch is selected from the group of matrix type patch, reservoir type patch, multi-laminate drug-in-adhesive type patch, and monolithic drug-in-adhesive type patch.

3. Transdermal patch according to at least one of the preceding claims, wherein the patch is a monolithic drug-in-adhesive type patch.

4. Transdermal patch according to at least one of the preceding claims, wherein the adhesive further comprises a component selected from the group of natural rubber; synthetic rubber; acrylic adhesive; polyvinylacetate; polydimethylsiloxane; and hydrogels, especially high molecular weight polyvinylpyrrolidone and oligomeric polyethylene oxide.

5. Transdermal patch according to claim 4, wherein the further adhesive is an acrylic adhesive.

6. Transdermal patch according to claim 5, wherein the acrylic adhesive comprises or consists of a polyacrylate.

7. Transdermal patch according to claim 6, wherein the polyacrylate is selected from the group consisting of polybutylacrylate, polmethylacrylate and poly-2-ethylhexylacrylate.

8. Transdermal patch according to at least one of claims 4 to 7, wherein the adhesive contains a crosslinker.

9. Transdermal patch according to at least one of the preceding claims, wherein the analgesic is buprenorphine or a pharmaceutically acceptable salt thereof.

10. Transdermal patch according to at least one of the preceding claims and especially claim 9, wherein the extracting agent of the aloe extract or the vehicle is a vegetable oil.

11. Transdermal patch according to claim 10, wherein the vegetable oil is a hydrogenated oil.

12. Transdermal patch according to claim 10 or 11, wherein the vegetable oil is soybean oil.

13. Transdermal patch according to at least one of the preceding claims, wherein the patch comprises another penetration agent in addition to the aloe composition.

14. Transdermal patch according to claim 13, wherein the additional penetration agent is selected from the group consisting of ethyl alcohol; isopropyl alcohol; octyl phenol; polyethylene glycol octylphenyl ether; oleic acid; polyethylene glycol (PEG), especially PEG 400; propylene glycol; n-decylmethyl sulfoxide; fatty acid esters, especially isopropyl myristate, methyl laurate, glycerol monooleate and propylene glycol monooleate; and N-methyl pyrrolidone.

15. Transdermal patch according to at least one of the preceding claims, wherein the composition comprises a preservative, especially a preservative selected from the group of alcohols, quaternary amines, organic acids, parabens and phenols.

16. Transdermal patch according to at least one of the preceding claims, wherein the backing comprises or consists of a material selected from the group consisting of polyolefin, polyester, polyvinylidene chloride, polyurethane, cotton or wool.

17. Transdermal patch according to claim 16, wherein the backing is a polyolefine foil.

18. Transdermal patch according to claim 17, wherein the foil has a thickness of 0.5 to 1.5 and especially 0.6 to 1.0 mm.

## Patentansprüche

1. Transdermales Pflaster, das aufweist:
• ein Opioid-Analgetikum aus der Phenanthrengruppe oder ein pharmazeutisch akzeptables Salz davon als wirksamen Bestandteil;
• eine Aloe-Zusammensetzung als transdermales Penetrationshilfsmittel; und
• eine Rückschicht, einen druckempfindlichen Klebstoff und eine Schutzfolie;
wobei der Klebstoff einen Kautschukklebstoff enthält oder daraus besteht, der unter einem Styrol-Butadien-Styrol-Blockcopolymer oder einem Styrol-Butadien-Blockcopolymer ausgewählt ist.

2. Transdermales Pflaster nach Anspruch 1, wobei das Pflaster unter den Pflastern vom Matrix-Typ, Pflastern vom Reservoir-Typ, Pflastern vom Typ der mehrschichtigen Drug-in-Adhesive-Pflaster und Pflastern vom Typ der monolithischen Drug-in-Adhesive-Pflaster ausgewählt ist.

3. Transdermales Pflaster nach zumindest einem der vorhergehenden Ansprüche, bei dem es sich um ein monolithisches Drug-in-Adhesive-Pflaster handelt.

4. Transdermales Pflaster nach zumindest einem der vorhergehenden Ansprüche, wobei der Klebstoff ferner eine Komponente enthält, die unter Naturkautschuken; Synthesekautschuken; Acrylklebstoffen; Polyvinylacetat; Polydimethylsiloxan; und Hydrogelen, insbesondere Polyvinylpyrrolidon mit hohem Molekulargewicht und oligomerem Polyethylenoxid ausgewählt ist.

5. Transdermales Pflaster nach Anspruch 4, wobei der weitere Klebstoff ein Acrylklebstoff ist.

6. Transdermales Pflaster nach Anspruch 5, wobei der Acrylklebstoff ein Polyacrylat enthält oder daraus besteht.

7. Transdermales Pflaster nach Anspruch 6, wobei das Polyacrylat unter Polybutylacrylat, Polymethylacrylat und Poly-2-ethylhexylacrylat ausgewählt ist.

8. Transdermales Pflaster nach einem Ansprüche 4 bis 7, wobei der Klebstoff ein Vernetzungsmittel enthält.

9. Transdermales Pflaster nach zumindest einem der vorhergehenden Ansprüche, wobei es sich bei dem Analgetikum um Buprenorphin oder ein pharmazeutisch akzeptables Salz davon handelt.

10. Transdermales Pflaster nach zumindest einem der vorhergehenden Ansprüche und insbesondere Anspruch 9, wobei das Extraktionsmittel für den Aloe-Extrakt oder der Trägerstoff ein Pflanzenöl ist.

11. Transdermales Pflaster nach Anspruch 10, wobei das Pflanzenöl ein hydriertes Öl ist.

12. Transdermales Pflaster nach Anspruch 10 oder 11, wobei es sich bei dem Pflanzenöl um Sojaöl handelt.

13. Transdermales Pflaster nach zumindest einem der vorhergehenden Ansprüche, wobei das Pflaster zusätzlich zu der Aloe-Zusammensetzung ein weiteres Penetrationshilfsmittel enthält.

14. Transdermales Pflaster nach Anspruch 13, wobei das weitere Penetrationshilfsmittel unter Ethanol; Isopropanol; Octylphenol; Polyethylenglycoloctylphenylether; Ölsäure; Polyethylenglycol (PEG), insbesondere PEG 400; Propylenglycol; n-Decylmethylsulfoxid; Fettsäureestern, insbesondere Isopropylmyristat, Methyllaurat, Glycerolmonooleat und Propylenglycolmonooleat; und N-Methylpyrrolidon ausgewählt ist.

15. Transdermales Pflaster nach zumindest einem der vorhergehenden Ansprüche, das die Zusammensetzung ein Konservierungsmittel enthält, insbesondere ein unter Alkoholen, quartären Aminen, organischen Säuren, Parabenen und Phenolen ausgewähltes Konservierungsmittel.

16. Transdermales Pflaster nach zumindest einem der vorhergehenden Ansprüche, wobei die Rückschicht ein Material enthält oder daraus besteht, das unter Polyolefin, Polyester, Polyvinylidenchlorid, Polyurethan, Baumwolle oder Wolle ausgewählt ist.

17. Transdermales Pflaster nach Anspruch 16, wobei die Rückschicht eine Polyolefinfolie ist.

18. Transdermales Pflaster nach Anspruch 17, wobei die Folie eine Dicke von 0,5 bis 1,5 mm und insbesondere 0,6 bis 1,0 mm aufweist.

## Revendications

1. Timbre transdermique comprenant :
- un analgésique opioïde du groupe du phénanthrène ou un sel pharmaceutiquement acceptable de celui-ci en tant que principe actif,
- une composition d'aloès en tant qu'agent de pénétration transdermique et
- une couche de revêtement, un adhésif autocollant et une doublure détachable, l'adhésif comprenant ou étant constitué d'un adhésif de caoutchouc sélectionné parmi un copolymère bloc de styrène-butadiène-styrène et un copolymère bloc de styrène-butadiène.

2. Timbre transdermique selon la revendication 1, dans lequel le timbre est sélectionné dans le groupe comprenant un timbre du type à matrice, un timbre du type à réservoir, un timbre du type multicouche avec un médicament incorporé dans l'adhésif et un timbre du type monolithique avec un médicament incorporé dans l'adhésif.

3. Timbre transdermique selon au moins l'une des revendications précédentes, dans lequel le timbre est un timbre du type monolithique avec un médicament incorporé dans l'adhésif.

4. Timbre transdermique selon au moins l'une des revendications précédentes, dans lequel l'adhésif comprend en outre un composant sélectionné dans le groupe comprenant du caoutchouc naturel ; du caoutchouc synthétique ; de l'adhésif acrylique ; du polyacétate de vinyle ; du polydiméthylsiloxane ; et des hydrogels, en particulier de la polyvinylpyrrolidone et du polyéthylène oxyde oligomérique à poids moléculaire élevé.

5. Timbre transdermique selon la revendication 4, dans lequel l'autre adhésif est un adhésif acrylique.

6. Timbre transdermique selon la revendication 5, dans lequel l'adhésif acrylique comprend ou est constitué par un polyacrylate.

7. Timbre transdermique selon la revendication 6, dans lequel le polyacrylate est sélectionné dans le groupe comprenant du polybutylacrylate, du polyméthylacrylate et du poly-2-éthylhexylacrylate.

8. Timbre transdermique selon au moins l'une des revendications 4 à 7, dans lequel l'adhésif contient un agent de réticulation.

9. Timbre transdermique selon au moins l'une des revendications précédentes, dans lequel l'analgésique est de la buprénorphine ou un sel pharmaceutiquement acceptable de celle-ci.

10. Timbre transdermique selon au moins l'une des revendications précédentes et en particulier la revendication 9, dans lequel l'agent d'extraction de l'extrait d'aloès ou de transmission est une huile végétale.

11. Timbre transdermique selon la revendication 10, dans lequel l'huile végétale est une huile hydrogénée.

12. Timbre transdermique selon la revendication 10 ou 11, dans lequel l'huile végétale est de l'huile de soja.

13. Timbre transdermique selon au moins l'une des revendications précédentes, dans lequel le timbre comprend un autre agent de pénétration en plus de la composition d'aloès.

14. Timbre transdermique selon la revendication 13, dans lequel l'agent de pénétration supplémentaire est sélectionné dans le groupe comprenant de l'alcool éthylique ; de l'alcool isopropylique ; de l'octyl phénol ; de l'éther octylphénylique de polyéthylène glycol ; de l'acide oléique ; du polyéthylène glycol (PEG), en particulier du PEG 400 ; du propylène glycol ; du sulfoxyde de n-décyle méthyle ; des esters d'acides gras, en particulier du myristate d'isopropyle, du laurate de méthyle, du monooléate de glycérol et du monooléate de propylène glycol ; et du N-méthyle pyrrolidone.

15. Timbre transdermique selon au moins l'une des revendications précédentes, dans lequel la composition comprend un agent de conservation, en particulier un agent de conservation sélectionné dans le groupe comprenant des alcools, des amines quaternaires, des acides organiques, des parabènes et des phénols.

16. Timbre transdermique selon au moins l'une des revendications précédentes, dans lequel le support comprend ou est constitué d'une matière sélectionnée dans le groupe comprenant de la polyoléfine, du polyester, du chlorure de polyvinylidène, du polyuréthane, du coton ou de la laine.

17. Timbre transdermique selon la revendication 16, dans lequel le support est une feuille de polyoléfine.

18. Timbre transdermique selon la revendication 17, dans lequel la feuille présente une épaisseur comprise entre 0.5 et 1.5 mm et, en particulier, entre 0.6 et 1.0 mm.
